# EUROPÄISCHE PATENTSCHRIFT

(11) **EP 1 042 986 B1**
(45) Veröffentlichungstag und Bekanntmachung des Hinweises auf die Patenterteilung: **29.08.2012**
(21) Anmeldenummer: 00106637.2
(22) Anmeldetag: 28.03.2000
(51) Int. Cl.: A61B 10/02, A61B 17/00

(54) **Biopsienadel, Nadelhalter und Biopsiesystem**
Biopsy needle, needle holder and biopsy system
Aiguille de biopsie, porte-aiguille et systeme de biopsie

(30) Priorität: 08.04.1999 DE 19915857
(43) Veröffentlichungstag der Anmeldung: 11.10.2000
(73) Patentinhaber: Pajunk GmbH & Co. KG Besitzverwaltung, 78187 Geisingen (DE)
(72) Erfinder: Pajunk, Heinrich, 78187 Geisingen/Kirchen-Hausen (DE); Pajunk, Horst, 78187 Geisingen (DE)
(74) Vertreter: Manitz, Finsterwald & Partner GbR

(56) Entgegenhaltungen:
- US-A- 5 163 947
- US-A- 5 236 334
- US-A- 5 388 589

## Beschreibung

Die vorliegende Erfindung betrifft eine Biopsienadel für die Entnahme von Gewebeproben zur Verwendung in einem Nadelhalter, insbesondere einer Biopsiepistole, gemäß dem Oberbegriff des Annspruchs 1.

Eine Biopsienadel mit den Merkmalen des Oberbegriffs des Anspruchs 1 ist aus der US 5,388,589 bekannt. US 5,236,334 beschreibt eine Biopsienadel mit einer Innennadel mit einem ersten Verbindungselement und einer Außenkanüle mit einem zweiten Verbindungselement, wobei die Innennadel über einen an dem ersten Verbindungselement angebrachten Arm an der Außenkanüle lösbar fixiert ist. US 5,163,947 beschreibt eine Biopsienadel mit einer Innennadel mit einem ersten Verbindungselement und einer Außenkanüle mit einem zweiten Verbindungselement, wobei ein Abstandshalter zwischen den Verbindungselementen lösbar mit dem zweiten Verbindungselement verbindbar ist.

Weiterhin betrifft die Erfindung ein Biopsiesystem mit einer Biopsienadel der eingangs genannten Art und einem Nadelhalter, im folgenden auch Biopsiepistole genannt, für die Biopsienadel.

Während früher Biopsienadeln und Nadelhalter oftmals eine einzige Einheit bildeten und nach ihrer Verwendung entweder als Einmalware entsorgt wurden oder als wiederverwendbare Ware sterilisiert wurden, ist bei heutigen Biopsiesystemen der Nadelhalter einerseits als wiederverwendbares Teil und die Biopsienadel als Einmalinstrument ausgebildet. Dies hat den Vorteil, daß der meist eine aufwendige Mechanik enthaltende vollautomatische Nadelhalter, der entsprechend hohe Herstellungskosten verursacht, praktisch beliebig oft verwendet werden kann, während die nur sehr schwer zu sterilisierende Biopsienadel, die relativ kostengünstig herstellbar ist, als Wegwerfprodukt ausgebildet werden kann.

Es hat sich gezeigt, daß bei einem solchen zweigeteilten System gerade das Einlegen und das Entnehmen der Biopsienadel in den bzw. aus dem Nadelhalter oftmals mit Schwierigkeiten verbunden ist. Zum einen ist der Abstand zwischen den beiden Verbindungselementen der Biopsienadel aufgrund der Verschiebbarkeit zwischen der Innennadel und der Außenkanüle veränderbar, so daß vor Einsetzen der Biopsienadel in den Nadelhalter die Verbindungselemente korrekt zueinander ausgerichtet werden müssen. Zum anderen können sich die Innennadel und die Außenkanüle auch bei der Entnahme der Biopsienadel aus dem Nadelhalter gegeneinander verschieben, wodurch die Ausnehmung mit der Gewebeprobe versehentlich freigelegt werden kann und die Gewebeprobe aus der Ausnehmung herausfallen bzw. zumindest durch die Umgebungsluft kontaminiert werden kann.

In den deutschen Gebrauchsmustern G 94 17 727.2 und G 94 14 728.0 sind eine Biopsienadel und ein Biopsiesystem beschrieben, bei dem die ' Verbindungselemente der Biopsienadel durch einen separaten, abnehmbaren Abstandshalter in einem vordefinierten Abstand zueinander eingestellt werden können. Diese Lösung hat jedoch mehrere Nachteile. Zum einen ist das Abnehmen und Aufsetzen des Abstandshalters auf die Verbindungselemente relativ umständlich und zum anderen ist sowohl das Einsetzen als auch die Entnahme der Biopsienadel aus dem Nadelhalter nur im gespannten Zustand möglich. Dies hat zum einen sicherheitstechnische Nachteile und zum anderen führt dies dazu, daß nach der Gewebeentnahme der Nadelhalter erneut gespannt werden muß, um die Biopsienadel aus dem Nadelhalter entnehmen zu können. Bei diesem erneuten Spannvorgang wird zunächst die Außenkanüle zurückgezogen und im zweiten Spannschritt die Innennadel in die Außenkanüle zurückgezogen, so daß zwischen diesen beiden Spannschritten die Gewebeprobe freiliegt und aus der Ausnehmung der Innennadel herausfallen kann.

Ein weiterer Nachteil besteht darin, daß der separate, abnehmbare Abstandshalter aufgrund seiner Abmessungen einen deutlichen Anteil an den Herstellungskosten der Biopsienadel besitzt und, da er wie die Biopsienadel selbst als Wegwerfprodukt konzipiert ist, dadurch die Kosten für eine entsprechend ausgebildete Biopsienadel deutlich erhöht werden.

Es ist eine Aufgabe der vorliegenden Erfindung, eine Biopsienadel sowie ein Biopsiesystem anzugeben, bei dem zum einen die Kosten für die Herstellung der als Wegwerfprödukte ausgebildeten Teile minimiert werden, > das Einsetzen sowie das Entnehmen der Biopsienadel in den bzw. aus dem Nadelhalter vereinfacht werden und gewährleistet ist, daß ein Kontaminieren bzw. versehentliches Herausfallen einer entnommenen Gewe- .. beprobe aus der Ausnehmung der Innennadel praktisch ausgeschlossen ist.

Der die Biopsienadel betreffende Teil der Aufgabe wird von einer Biopsienadel mit den Merkmalen des Anspruchs 1 gelöst.

Durch die Ausbildung eines Fixierelements unmittelbar an dem zweiten Verbindungselement der Biopsienadel kann auf einfache und kostengünstige Weise sowohl erreicht werden, daß der Abstand zwischen dem ersten und dem zweiten Verbindungselement vor Einsetzen der Biopsienadel unmittelbar nach Fertigung eingestellt und fixiert werden kann als auch daß nach Durchführen der Biopsieentnahme die Innennadel und die Außenkanüle über das Fixierelement unmittelbar gegeneinander fixiert werden können, ohne daß vorher die Biopsienadel aus dem Nadelhalter entnommen werden müßte oder der Nadelhalter nochmals neu gespannt werden müßte. Da sich zu diesem Zeitpunkt das entnommene Gewebematerial sicher innerhalb der Außenkanüle befindet, ist somit ein versehentliches Herausfallen der Gewebeprobe aus der Ausnehmung der Innennadel aufgeschlossen.

Die Biopsienadel kann somit unmittelbar nach dem Fixieren aus dem Nadelhalter entnommen und beispielsweise in ein Labor verbracht werden.

Durch die Anordnung des Fixierelements an dem an der Außenkanüle vorgesehenen zweiten Verbindungselement ist es nicht erforderlich, daß zur Verhinderung einer Verschiebung von Innennadel gegenüber Außenkanüle ein zwischen den beiden Verbindungselementen wirkender Abstandshalter eingesetzt wird. Da das zweite Verbindungselement unmittelbar an der Außenkanüle vorgesehen ist und auch die Innennadel daher durch das zweite Verbindungselement hindurchlaufen bzw. unmittelbar an diesem vorbeigeführt sein muß, kann in diesem Bereich eine Einwirkung sowohl auf die Innennadel als auch auf die Außenkanüle erfolgen. Auf diese Weise ist somit zum einen eine deutliche Materialersparnis gegenüber einer Lösung mit Abstandshalter erzielbar und zum anderen kann das Fixierelement so in das zweite Verbindungselement integriert werden, daß aufgrund des minimal zusätzlichen Platzbedarfs die Funktionalität des Nadelhalters nicht beeinträchtigt ist.

Die Innennadel und die Außenkanüle und/oder die Innennadel und das zweite Verbindungselement sind über das Fixierelement miteinander verklemmbar. Durch die erfindungsgemäße Verklemmung wird eine leicht wieder lösbare Fixierung geschaffen, die keinerlei Spuren einer äußeren Einwirkung hinterläßt. Weiterhin sind bei einer Verklemmung an der Innennadel und der Außenkanüle selbst keinerlei Veränderungen vorzunehmen, so daß handelsübliche Außenkanülen und Innennadeln verwendet werden können.

Nach einer bevorzugten Ausführungsform der Erfindung ist das Fixierelement integral mit bzw. nicht abnehmbar von dem zweiten Verbindungselement ausgebildet. Bevorzugt ist das Fixierelement dabei mit dem zweiten Verbindungselement verklebt.

Durch die integrale bzw. nicht abnehmbare Ausbildung wird eine sehr kompakte Bauform geschaffen, die sich äußerlich von einer bekannten Biopsienadel nur geringfügig unterscheidet und somit neben den geringen Herstellungskosten auch die Akzeptanz der Anwender erfährt. Gerade dadurch, daß kein zusätzliches Element erforderlich ist, kann die Handhabung vereinfacht werden, da jedes zusätzliche Element die Wahrscheinlichkeit einer Fehlbedienung durch fehlerhaftes Anbringen erhöht. Weiterhin kann ein separates Teil während der Biopsieentnahme verlorengehen, so daß ein Entnehmen der Biopsienadel aus dem Nadelhalter in diesem Fall erschwert oder völlig unmöglich gemacht wird.

Bevorzugt ist zur Verklemmung von Innennadel und Außenkanüle die Innennadel durch die Klemmeinheit gegen die Innenwand der Außenkanüle preßbar. Dazu umfaßt das Fixierelement vorteilhaft eine Drehbetätigung, die um eine im wesentlichen senkrecht zur Längsachse der Außenkanüle verlaufende Drehachse verdrehbar ist, wodurch eine mit der Drehbetätigung verbundene Klemmeinheit entlang der Drehachse verschiebbar ist.

Eine Drehbetätigung hat den Vorteil, daß zum einen ein geringer Platzbedarf erforderlich ist und zum anderen vom Anwender intuitiv zum Fixieren die Drehbetätigung in Uhrzeigerrichtung und zum Lösen der Fixierung die Drehbetätigung gegen Uhrzeigerrichtung verdreht wird. Durch die entlang der Drehachse verschiebbare Klemmeinheit wird beispielsweise die Außenkanüle bereichsweise ebenfalls entlang der Drehachse, d.h. senkrecht zu ihrer Längserstreckung, verschoben, wodurch eine Verklemmung von Außenkanüle und Innennadel erfolgt.

Vorteilhaft besitzt die Klemmeinheit dazu eine Durchgangsöffnung, insbesondere eine Bohrung zum Durchführen der Innennadel und ist über ein Gewinde mit der Drehbetätigung verbunden. Der in der Durchgangsöffnung angeordnete Bereich der Innennadel wird bei einer Verschiebung der Klemmeinheit senkrecht zur Längserstreckung der Innennadel mitgenommen, so daß die gewünschte Querverschiebung zwischen Innennadel und Außenkanüle erzielt wird. Die Klemmeinheit kann grundsätzlich auch ohne Durchgangsöffnung ausgebildet sein und mit einer Angriffsfläche beispielsweise an der Außenseite der Innennadel anliegen, so daß bei einem Verdrehen der Drehbetätigung und dem damit verbundenen Verschieben der Klemmeinheit ebenfalls eine Querverschiebung der Innennadel erreicht wird.

Grundsätzlich kann anstelle einer Drehbetätigung auch eine andere Art von Betätigungseinrichtung, beispielsweise ein Schiebeelement oder ein Druckknopf vorgesehen sein.

Nach einer weiteren bevorzugten Ausführungsform der Erfindung ist die Drehbetätigung zwischen zwei Anschlägen verstellbar, wobei der eine Anschlag die Fixierstellung und der andere Anschlag die Freigabestellung definiert. Auf diese Weise wird die Bedienung einer erfindungsgemäßen Biopsienadel weiter vereinfacht, da zum Umschalten von der Fixier- in die Freigabestellung die Drehbetätigung jeweils vollständig bis zu einem der Endanschläge verdreht werden kann.

Nach einer weiteren vorteilhaften Ausführungsform der Erfindung umfaßt die Drehbetätigung eine länglich ausgebildete Handhabe, die in der Fixierstellung von Innennadel und Außenkanüle im wesentlichen parallel zur Außenkante verläuft. Bevorzugt verläuft die Handhabe dementsprechend in der Freigabestellung im wesentlichen senkrecht zur Außenkanüle.

Die Ausbildung einer länglichen Handhabe besitzt mehrere Vorteile. Zum einen gibt die parallel zur Außenkanüle verlaufende Handhabe dem Anwender bereits intuitiv den Hinweis, daß in dieser Stellung die Innennadel und die Außenkanüle gegeneinander verschiebbar sind, während die senkrecht zur Außenkanüle verlaufende Handhabe intuitiv die Fixierstellung kennzeichnet. Zum anderen kann, wie im weiteren dargestellt wird, eine derart ausgebildete Handhabe zum einen als Spannungsanzeige und zum anderen als Einsetzhilfe dienen. Dazu kann die Handhabe bevorzugt einen Bereich, insbesondere einen Ansatz, aufweisen, der zum verschiebbaren Einsetzen in eine Längsausnehmung eines Gehäuseteils, insbesondere eines Deckels, des Nadelhalters ausgebildet ist. Dieser Bereich bzw. Ansatz ist dabei bevorzugt länglich ausgebildet und erstreckt sich insbesondere in Längsrichtung der Handhabe.

Durch diese Ausführungsform ist gewährleistet, daß sich der Deckel des Gehäuseteils des Nadelhalters nur dann schließen läßt, wenn vorher die Handhabe in die Freigabestellung verdreht wurde. Nur in dieser Stellung kann der länglich ausgebildete Bereich der Handhabe in die Längsausnehmung des sich schließenden Deckels eintreten, so daß dieser vollständig geschlossen werden kann.

Befindet sich die Handhabe in der Fixierstellung, so ist ein Eintreten des länglichen Bereichs der Handhabe in die Längsausnehmung nicht möglich, da diese senkrecht zueinander angeordnet sind. Somit kann in diesem Fall der Deckel nicht geschlossen werden.

Ist die Längsausnehmung als Längsdurchbrechung ausgebildet, so ist der längliche Bereich der Handhabe bei geschlossenem Deckel von außen sichtbar und kann als Anzeige für den Spannungszustand des Nadelhalters verwendet werden.

Nach einer weiteren vorteilhaften Ausführungsform der Erfindung sind das erste und/oder das zweite Verbindungselement zum Verrasten mit dem ersten bzw. dem zweiten Kopplungselement ausgebildet. Dazu können beispielsweise federbeaufschlagte Kugelelemente sowie entsprechende Ausnehmungen an den Verbindungselementen bzw. den Kopplungselementen vorgesehen sein. Durch die Verrastung der Verbindungs- und Kopplungselemente wird erreicht, daß bereits nach Einsetzen der Biopsienadel in den Nadelhalter eine gewisse Fixierung vorhanden ist, so daß bereits vor Schließen des Deckels die Biopsienadel nicht versehentlich aus dem Nadelhalter herausfallen kann. Dies ist insbesondere wesentlich, da bei einem entsprechenden Herausfallen die Biopsienadel mit großer Wahrscheinlichkeit kontaminiert wäre oder insbesondere deren Spitze beschädigt wäre, so daß eine neue Biopsienadel verwendet werden müßte.

Der das Biopsiesystem betreffende Teil der Aufgabe wird von einem Biopsiesystem mit den Merkmalen des Anspruchs 9 gelöst.

Bei diesem Biopsiesystem ist gewährleistet, daß sowohl beim Einsetzen als auch beim Entnehmen der Nadel der Nadelhalter ungespannt ist und somit keine beispielsweise durch Fehlbedienung bedingte Auslösung möglich ist, durch die eine Verletzung des Patienten oder der Bedienungsperson erfolgen könnte. Weiterhin wird beim Spannen des Nadelhalters das in der Ausnehmung befindliche Gewebe nur für einen minimalen Zeitraum freigelegt.

Vorteilhaft sind in einem ersten Schritt zum Teilspannen des Nadelhalters das zweite Kopplungselement mit der Außenkanüle und in einem darauffolgenden zweiten Schritt zum vollständigen Spannen des Nadelhalters das erste Kopplungselement mit der Innennadel gegen die Federvorspannung verschiebbar. Dadurch sind hintereinanderliegende Mehrfachentnahmen von Gewebeproben möglich, da nach der Teilspannung das entnommene Gewebe aus der Ausnehmung in einen Behälter gegeben werden kann und anschließend nach einem weiteren Spannvorgang der Nadelhalter wieder vollständig für die nächste Entnahme gespannt ist.

Es ist jedoch grundsätzlich auch möglich, den Nadelhalter so auszubilden, daß in einem ersten Schritt zum Teilspannen des Nadelhalters das erste Kopplungselement und in einem darauffolgenden zweiten Schritt zum vollständigen Spannen des Nadelhalters das zweite Kopplungselement gegen die Federvorspannung verschoben wird. In diesem Fall sind zwar keine Mehrfachentnahmen von Gewebeproben möglich, es ist jedoch gewährleistet, daß das entnommene Gewebe in keinem Fall durch Spannen des Nadelhalters freigelegt wird, so daß erst nach Entnahme der Nadel aus dem Nadelhalter ein Freigeben der Gewebeprobe erfolgen kann.

Nach einer weiteren bevorzugten Ausführungsform der Erfindung ist der Verschiebeweg des ersten und des zweiten Kopplungselements und damit die Eindringtiefe der Biopsienadel kontinuierlich einstellbar. Vorteilhaft sind zur Begrenzung der Verschiebewege für das erste und das zweite Kopplungselement jeweils Begrenzungsanschläge vorgesehen, wobei die Begrenzungsanschläge insbesondere über ein Schraubgewinde kontinuierlich parallel zur Verschieberichtung der Kopplungselemente verstellbar sind.

Durch die kontinuierliche Einstellbarkeit der Eindringtiefe der Biopsienadel kann die Flexibilität eines erfindungsgemäß ausgebildeten Biopsiesystems weiter verbessert werden, wobei durch die konstruktive Ausgestaltung über eine Schraubverstellung das Biopsiesystem sehr einfach und kostengünstig herstellbar ist.

Nach einer weiteren bevorzugten Ausführungsform der Erfindung sind im Bereich zwischen den Kopplungselementen und den Begrenzungsanschlägen, insbesondere an den Begrenzungsanschlägen, insbesondere elastische Dämpfungselemente zur Aufprall- und/oder Schalldämpfung vorgesehen. Durch die Dämpfungselemente wird zum einen die Geräuschentwicklung beim Aufprallen der Kopplungselemente auf den Begrenzungsanschlägen verringert, was sowohl beim Patienten als auch bei dem behandelnden Arzt störende Reflexbewegungen verringert oder verhindert. Weiterhin werden durch die elastischen Dämpfungselemente auch die Erschütterungen beim Aufschlagen der Kopplungselemente auf die Begrenzungsanschläge gedämpft.

Nach einer weiteren vorteilhaften Ausführungsform der Erfindung ist zum Entspannen des Nadelhalters und damit zum Einschießen der Biopsienadel ein Druckknopf an der der Austrittsstelle der Biopsienadel gegenüberliegenden Seite des Nadelhalters angeordnet ist. Durch die Anordnung eines Druckknopfes an der rückwärtigen Seite des Nadelhalters wird eine sehr gute Bedienbarkeit zum Einschießen der Biopsienadel erzielt. Gleichzeitig wird durch diese Anordnung sowohl eine links- als auch eine rechtshändige Bedienung möglich.

Weitere vorteilhafte Ausführungsformen der Erfindung sind in den Unteransprüchen angegeben.

Die Erfindung wird nachfolgend anhand eines Ausführungsbeispiels unter Bezugnahme auf die Zeichnungen näher beschrieben; in diesen zeigen:
- Figur 1: eine erfindungsgemäß ausgebildete Biopsienadel in der Fixierstellung,
- Figur 2: einen Längsschnitt durch einen Teil der Biopsienadel nach Figur 1 in der Freigabestellung,
- Figur 3: einen teilweise aufgerissen dargestellten Nadelhalter im vollständig gespannten Zustand beim Einsetzen einer erfindungsgemäß ausgebildeten Biopsienadel,
- Figur 4: der Nadelhalter nach Figur 3 im entspannten Zustand beim Entnehmen einer Biopsienadel und
- Figur 5: der Nadelhalter gemäß den Figuren 3 und 4 im geschlossenen Zustand.

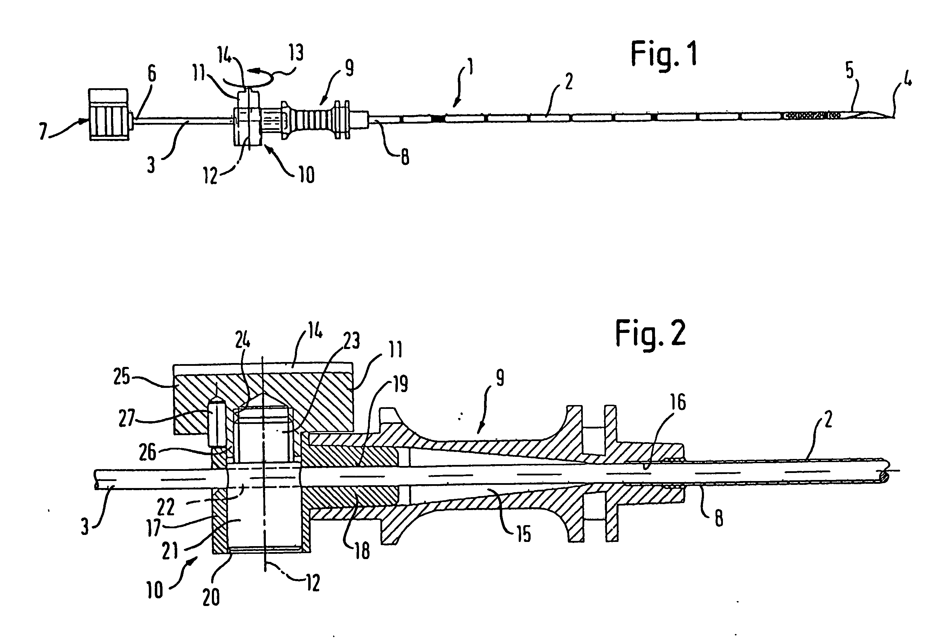

Die in Fig. 1 dargestellte Biopsienadel 1 umfaßt eine Außenkanüle 2, in der verschieblich gelagert eine Innennadel 3 angeordnet ist.

An dem distalen Ende der Innennadel 3 ist eine Spitze 4 ausgebildet, die aus dem distalen Ende 5 der Außenkanüle 2 herausragt, während an dem proximalen Ende 6 der Innennadel 3 ein erstes Verbindungselement 7 ausgebildet ist.

An dem proximalen Ende 8 der Außenkanüle 2 ist ein zweites Verbindungselement 9 vorgesehen, an dessen proximalen Ende sich ein Fixierelement 10 anschließt, das mit dem zweiten Verbindungselement 9 beispielsweise verklebt ist.

Das Fixierelement 10 umfaßt eine Drehbetätigung 11, die um eine senkrecht zur Außenkanüle 2 verlaufende Drehachse 12 verdrehbar ist, wie es durch einen Pfeil 13 angedeutet ist.

An der Oberseite der Drehbetätigung 11 ist ein länglicher Ansatz 14 ausgebildet, der sich beispielsweise über die gesamte Breite der Drehbetätigung 11 erstreckt.

Aus der in Fig. 2 dargestellten Schnittdarstellung ist der Aufbau des zweiten Verbindungselements 9 sowie des Fixierelements 10 deutlicher erkennbar.

Das zweite Verbindungselement 9 besitzt eine Durchgangsöffnung 15, die sich von einem Durchmesser, der dem Außendurchmesser der Außenkanüle 2 entspricht in Richtung zu dem Fixierelement 10 zu einem größeren Durchmesser hin aufweitet. Die Außenkanüle 2 ist in das distale Ende 16 der Durchgangsöffnung 15 eingesetzt und in dieser befestigt, beispielsweise verklebt, während sich die Innennadel 3 vollständig durch die Durchgangsöffnung 15 hindurch und in die Außenkanüle 3 hinein erstreckt.

Das Fixierelement 10 umfaßt einen Basiskörper 17 mit einem seitlichen Ansatz 18 der eine Durchgangsöffnung 19 zur Durchführung der Innennadel 3 besitzt und auf den das zweite Verbindungselement 9 aufgesteckt ist. Bevorzugt ist das zweite Verbindungselement 9 dabei mit dem seitlichen Ansatz 18 verklebt oder auf eine sonstige Weise nicht abnehmbar mit diesem verbunden. Grundsätzlich können der Basiskörper 17 und das zweite Verbindungselement 9 auch einstückig ausgebildet sein.

Der Basiskörper 17 umfaßt einen Hohlraum 20, in dem ein Klemmelement 21 entlang der Drehachse 12 verschiebbar angeordnet ist. Das Klemmelement 21 besitzt eine Durchgangsöffnung 22, die fluchtend mit der Durchgangsöffnung 15 des zweiten Verbindungselements 9 angeordnet ist und durch die die Innennadel 3 geführt ist.

An der Oberseite des Klemmelements 21 ist ein Gewindeabschnitt 23 ausgebildet, der in ein entsprechendes Innengewinde 24 der Drehbetätigung 11 eingeschraubt ist.

Die Drehbetätigung 11 umfaßt eine länglich ausgebildete Handhabe 25, an deren Unterseite ein sich in den Hohlraum 20 hinein erstreckender, ringförmiger Ansatz 26 ausgebildet ist, an dessen Innenseite das Innengewinde 24 vorgesehen ist.

Durch Verdrehen der Drehbetätigung 11 über die Handhabe 25 wird das Klemmelement 21 entlang der Drehachse 12 verschoben, so daß die durch die Durchgangsöffnung 22 verlaufende Innennadel 3 ebenfalls entlang der Drehachse 12 verschoben wird, wodurch eine Verklemmung zwischen der Innennadel 3 und dem Basiskörper 17 erfolgt.

In Fig. 2 erstreckt sich die länglich ausgebildete Handhabe 25 parallel zur Längserstreckung der Außenkanüle 2, so daß sich das Fixierelement 10 in seiner Freigabestellung befindet, in der die Innennadel 3 im wesentlichen frei verschiebbar in den Durchgangsbohrungen 22, 19, 15 sowie der Außenkanüle 2 angeordnet ist. Dabei ist die Drehbetätigung 11 in dieser Stellung über ein in Figur 2 angedeutetes Fixierelement, beispielsweise eine federbeaufschlagte Kugelverrastung 27 definiert festlegbar.

Bevorzugt ist die Drehbetätigung 11 auch in der Fig. 1 dargestellten Fixierstellung, in der die längliche Drehbetätigung 11 senkrecht zur Längserstreckung der Außenkanüle 2 angeordnet ist und in der die Innennadel 3 durch das Klemmelement 21 unverschiebbar fixiert ist, über die Kugelverrastung 27 definiert festlegbar.

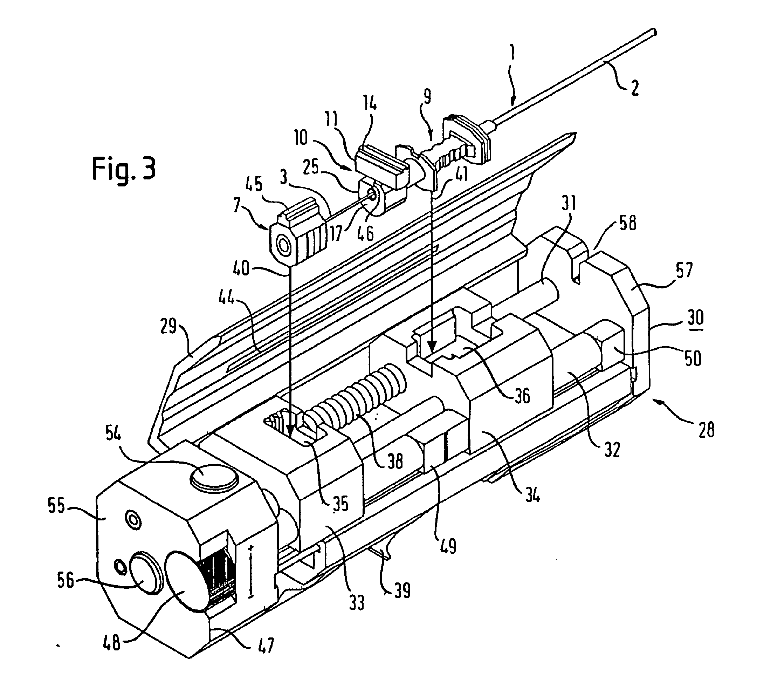

Fig. 3 zeigt einen Nadelhalter 28, der einen aufklappbaren Deckel 29 sowie ein Gehäuse 30 umfaßt, wobei zur besseren Darstellung der innerhalb des Gehäuses 30 liegenden Elemente die vordere Seitenwand des Gehäuses 30 nicht dargestellt ist.

In dem Gehäuse 30 sind entlang von Führungsstangen 31, 32 ein erstes und ein zweites schlittenförmiges Kopplungselement 33, 34 vorgesehen, die jeweils an ihren Oberseiten Aufnahmeöffnungen 35, 36 zum Einsetzen des ersten bzw. zweiten Verbindungselementes 7, 9 besitzen.

Die Aufnahmeöffnungen 35, 36 sind dabei im wesentlichen komplementär zu der Form des ersten bzw. zweiten Verbindungselements 7, 9 ausgebildet, so daß nach Einsetzen des ersten bzw. zweiten Verbindungselementes in die Aufnahmeöffnung 35 bzw. 36 eine in Richtung der Führungsstangen 31, 32 formschlüssige Verbindung besteht.

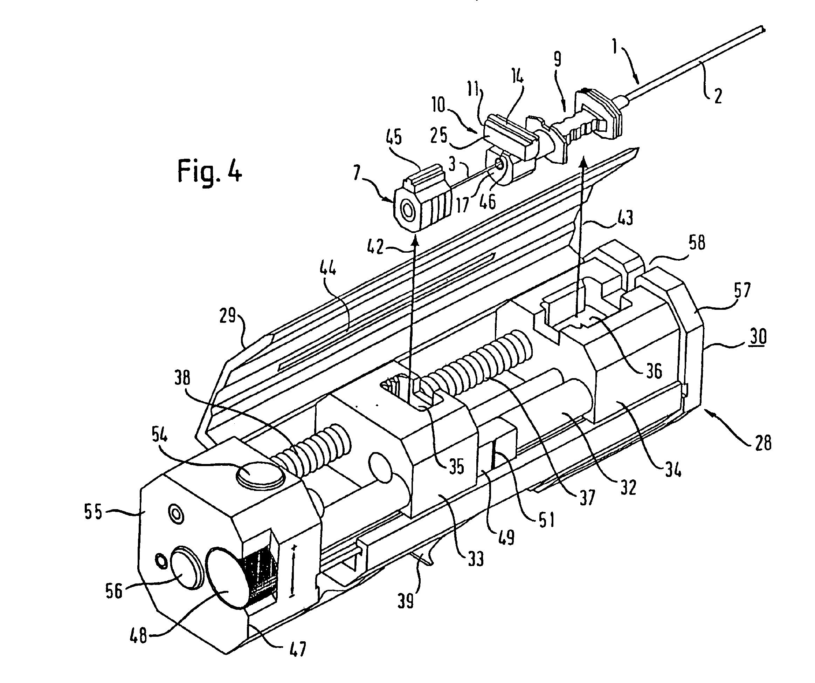

Die schlittenförmigen Kopplungselemente 33, 34 sind entlang der Führungsstangen 31, 32 gegen die Federkraft von Federn 37, 38 (siehe Fig. 4) durch Betätigen eines Schiebers 39 verschiebbar, wobei nach einmaligem Zurückschieben des Schiebers 39 zum Teilspannen des Nadelhalters zunächst das zweite Kopplungselement 34 und nach einen wiederholten Zurückschieben des Schiebers 39 zum vollständigen Spannen des Nadelhalters 28 das erste Kopplungselement 33 verschoben wird, so daß das erste und das zweite Kopplungselement 33, 34 nach zweimaligem Betätigen des Schiebers 39 die in Fig. 3 dargestellten Positionen einnehmen.

Wie in Fig. 3 durch zwei Pfeile 40, 41 angedeutet ist, ist die Biopsienadel 1 in ihrem fixierten Zustand, d.h. bei quergestellter Handhabe 25 mit dem ersten und dem zweiten Verbindungselement 7, 9 in die Aufnahmeöffnungen 35, 36 des ersten bzw. zweiten Kopplungselements 33, 34 einsetzbar, auch wenn sich der Nadelhalter 1 in seinem vollständigen gespannten Zustand befindet.

Bevorzugt erfolgt das Einsetzen jedoch in dem vollständig entspannten Zustand des Nadelhalters 1, wie er in Fig. 4 dargestellt ist. Dabei ist in Fig. 4 durch Pfeile 42, 43 angedeutet, daß die Biopsienadel 1 im entspannten Zustand des Nadelhalters 1 auch aus diesem entnommen werden kann.

In dem Deckel 29 des Nadelhalters 28 ist eine schlitzförmige Längsausnehmung 44 ausgebildet, in die nach Einsetzen der Biopsienadel 1, Verdrehen der Handhabe 25, bis sich diese in der Freigabestellung befindet, und anschließendem Schließen des Deckels 29 der längliche Ansatz 14 sowie ein an der Oberseite des ersten Verbindungselements 7 ausgebildeter weiterer länglicher Ansatz 45 hineinragen.

Die Ansätze 14 und 45 bilden auf diese Weise Spannungs-Anzeigelemente, die durch die Längsausnehmung 44 hindurch nach außen den jeweiligen Spannungszustand des Nadelhalters 28 anzeigen.

In Fig. 3 ist weiterhin zu erkennen, daß an dem Basiskörper 17 ein Anschlag 46 ausgebildet ist, durch den eine Begrenzung der Verdrehbarkeit der Handhabe 25 erfolgt. Durch einen weiteren in Fig. 3 nicht sichtbaren Anschlag ist zusammen mit dem Anschlag 46 gewährleistet, daß die Handhabe 25 definiert von der Fixierstellung in die Freigabestellung verdrehbar ist.

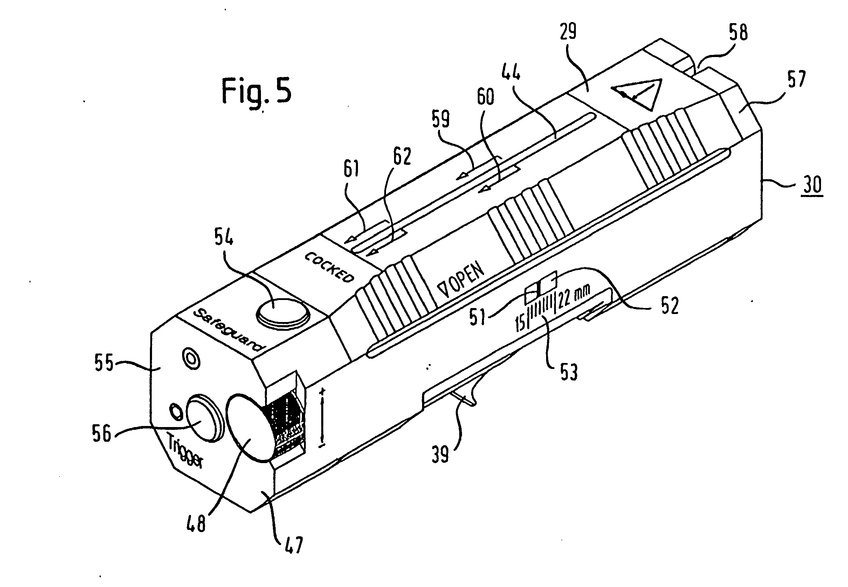

An dem rückwärtigen Ende 47 des Gehäuses 30 des Nadelhalters 28 ist eine Rändelschraube 48 ausgebildet, über die die Eindringtiefe der Biopsienadel 1 kontinuierlich einstellbar ist. Dazu sind an der Führungsstange 32 Begrenzungsanschläge 49, 50 vorgesehen, die durch Betätigen der Rändelschraube 48 über ein Schraubgewinde entlang der Führungsstange 32 verschiebbar sind. Weiterhin ist an dem Begrenzungsanschlag 49 eine Markierung 51 vorgesehen, die über ein Sichtfenster 52 (Fig. 5) von außen erkennbar ist, so daß anhand einer an dem Gehäuse vorgesehenen Skalierung 53 (Fig. 5) die jeweils eingestellte Eindringtiefe der Biopsienadel 1 ablesbar ist.

An der Oberseite des Gehäuses 30 ist ein Entsicherungsknopf 54 vorgesehen, durch dessen Drücken der vollständig gespannte Nadelhalter 28 in einen ungesicherten, schußbereiten Zustand überführt wird.

Zum Abschießen der Biopsienadel 1 ist an der stirnseitigen Rückwand 55 des Gehäuses 30 ein Druckknopf 56 vorgesehen, der einer in der stirnseitigen Wand 57 des Gehäuses 30 ausgebildeten Austrittsöffnung 58 der Biopsienadel gegenüberliegt.

Die erfindungsgemäß ausgebildete Vorrichtung wird wie folgt verwendet:

Die Biopsienadel 1 wird in ihrem fixierten Zustand in den entspannten Nadelhalter 28 eingesetzt. Dabei kann das erste und das zweite Verbindungselement 7, 9 durch nicht dargestellte Rastelemente, beispielsweise mit federbeaufschlagten Kugeln, in den Aufnahmeöffnungen 35, 35, in denen beispielsweise entsprechend ausgebildete Rastgegenelemente zum Beispiel in Form von Vertiefungen ausgebildet sind, einrasten. Auf diese Weise ist gewährleistet, daß auch bei einem Umdrehen des Nadelhalters 28 die eingesetzte Biopsienadel 1 nicht aus dem Nadelhalter 28 herausfallen kann. Die Biopsienadel 1 ist dabei durch das Fixierelement 10 so gesichert, daß die Innennadel 3, und die Außenkanüle 2 nicht gegeneinander verschiebbar sind.

Nach Einsetzen der Biopsienadel 1 wird deren Fixierung durch Verdrehen der Handhabe 25 gelöst, wobei die Handhabe 25 soweit gedreht wird, daß sie parallel zur Längserstreckung der Außenkanüle 2 ausgerichtet ist. Auf diese Weise ist gewährleistet daß ein Schließen des Deckels 29 möglich ist, da nur bei einer derartigen Stellung der Handhabe 25 der Ansatz 14 in die Längsausnehmung 44 eingreifen kann. Wird vergessen die Biopsienadel 1 durch Verdrehen der Handhabe zu entsichern, so läßt sich der Dekkel 29 nicht vollständig schließen.

Nach Schließen des Deckels 29 wird zunächst durch einmaliges Zurückziehen des Schiebers 39 das zweite Kopplungselement 34 und mit diesem zusammen die Außenkanüle 2 zurückgezogen, so daß sich der Nadelhalter 28 in einem teilgespannten Zustand befindet. Dieser teilgespannte Zustand ist von außerhalb des Gehäuses durch die Position des Ansatzes 14 in der Längsausnehmung 44 erkennbar. Gemäß Fig. 5 ist diese Position an der Außenseite des Deckels 29 durch zwei Pfeile 59, 60 markiert.

Nach erneutem Zurückziehen des Schiebers 39 wird auch das erste Kopplungselement 33 und mit diesem zusammen die Innennadel 3 zurückgezogen, woraufhin sich der Nadelhalter 28 in seinem vollständig gespannten Zustand befindet. Dieser ist anhand der Position des Ansatzes 45 in der Längsausnehmung 44 erkennbar. Auch diese Stellung ist, wie in Fig. 5 ersichtlich, durch an der Außenseite des Deckels 29 vorgesehene Pfeile 61, 62 erkennbar.

Die Biopsienadel 1 des auf diese Weise vollständig gespannten Nadelhalters 28 wird nun in die gewünschte Position gebracht, woraufhin durch Betätigen des Entsicherungsknopfes 54 und anschließendes Drücken des Druckknopfes 56 zunächst die Innennadel 3 nach vorne verschoben wird, so daß das zu entnehmende Gewebe in einer nicht dargestellte Ausnehmung im Bereich des distalen Endes der Innennadel 3 zu liegen kommt. Unmittelbar danach wird automatisch die Außenkanüle 2 nach vorne verschoben, so daß das in der Ausnehmung befindliche Gewebe vollständig innerhalb der Außenkanüle 2 zu liegen kommt.

Nachdem die Biopsienadel 1 zusammen mit dem Nadelhalter 28 wieder vollständig aus dem zu untersuchenden Gewebe herausgezogen wurde, ist somit das entnommene Gewebe sicher innerhalb der Außenkanüle 2 untergebracht, so daß keine Kontaminierung des Gewebes erfolgen kann.

Sollen mit der gleichen Biopsienadel 1 mehrere Gewebeproben hintereinander entnommen werden, so wird zunächst durch Zurückziehen des Schiebers 39 die Außenkanüle 2 zurückgezogen, wodurch das in der Ausnehmung liegende Gewebe zugänglich wird und in ein entsprechendes Behältnis gegeben werden kann. Durch erneutes Zurückziehen des Schiebers 39 ist dann der Nadelhalter 28 für die nächste Gewebeentnahme vorbereitet.

Soll hingegen nur eine einzige Gewebeprobe entnommen werden, so kann nach dieser Gewebeentnahme der Deckel 29 des Nadelhalters 29 geöffnet werden und die Handhabe 25 noch bei in dem Nadelhalter 28 befindlicher Biopsienadel 1 in die Fixierstellung gedreht werden. Auf diese Weise ist gewährleistet, daß beim Entnehmen der Biopsienadel 1 aus dem Gehäuse 30 nicht versehentlich die Innennadel 3 und die Außenkanüle 2 ineinander verschoben werden, wodurch das entnommene Gewebe aus der Ausnehmung herausfallen bzw. kontaminiert werden könnte.

Nach Fixierung der Biopsienadel 1 kann diese aus dem Nadelhalter 28 entnommen und beispielsweise in ein Labor gebracht werden. Dort kann durch Entriegeln des Fixierelementes 10 und anschließendes Vorschieben der Innennadel 3 das entnommene Gewebe zu weiteren Untersuchungszwecken freigegeben werden.

### Bezugszeichenliste

- 1: Biopsienadel
- 2: Außenkanüle
- 3: Innennadel
- 4: Spitze
- 5: distales Ende der Außenkanüle
- 6: proximales Ende der Innennadel
- 7: erstes Verbindungselement
- 8: proximales Ende der Außenkanüle
- 9: zweites Verbindungselement
- 10: Fixierelement
- 11: Drehbetätigung
- 12: Drehachse
- 13: Pfeil
- 14: Ansatz
- 15: Durchgangsöffnung
- 16: distales Endes der Durchgangsöffnung
- 17: Basiskörper
- 18: seitlicher Ansatz
- 19: Durchgangsöffnung
- 20: Hohlraum
- 21: Klemmelement
- 22: Durchgangsöffnung
- 23: Gewindeabschnitt
- 24: Innengewinde
- 25: Handhabe
- 26: ringförmiger Ansatz
- 27: Kugelverrastung
- 28: Nadelhalter
- 29: Deckel
- 30: Gehäuse
- 31: Führungsstange
- 32: Führungsstange
- 33: erstes Kopplungselement
- 34: zweites Kopplungselement
- 35: Aufnahmeöffnung
- 36: Aufnahmeöffnung
- 37: Feder
- 38: Feder
- 39: Schieber
- 40: Pfeil
- 41: Pfeil
- 42: Pfeil
- 43: Pfeil
- 44: Längsausnehmung
- 45: Ansatz
- 46: Anschlag
- 47: rückwärtiges Ende des Nadelhalters
- 48: Rendelschraube
- 49: Begrenzungsanschlag
- 50: Begrenzungsanschlag
- 51: Markierung
- 52: Sichtfenster
- 53: Skalierung
- 54: Entsicherungsknopf
- 55: Rückwand
- 56: Druckknopf
- 57: Wand
- 58: Austrittsöffnung
- 59: Pfeil
- 60: Pfeil
- 61: Pfeil
- 62: Pfeil

## Patentansprüche

1. Biopsienadel für die Entnahme von Gewebeproben zur Verwendung in einem Nadelhalter (28), insbesondere einer Biopsiepistole, mit einer Innennadel (3), die im Bereich ihres distalen Endes eine Ausnehmung für die Aufnahme der zu entnehmenden Gewebeprobe und im Bereich ihres proximalen Endes (6) ein erstes Verbindungselement (7) zum Verbinden mit einem ersten Kopplungselement (33) des Nadelhalters (28) aufweist, und mit einer Außenkanüle (2), in der die Innennadel (3) längsverschiebbar gelagert ist, und die im Bereich ihres proximalen Endes (8) ein zweites Verbindungselement (9) zum Verbinden mit einem zweiten Kopplungselement (34) des Nadelhalters (28) aufweist, welches zweite Verbindungselement (9) ein Fixierelement (10) umfaßt, das zwischen einer Fixier- und einer Freigabestellung verstellbar ist, wobei in der Freigabestellung die Innennadel (3) und die Außenkanüle (2) gegeneinander längsverschiebbar sind und in der Fixierstellung die Innennadel (3) und die Außenkanüle (2) gegeneinander unverschiebbar fixiert sind,
**dadurch gekennzeichnet.**
**daß** die Innennadel (3) und die Außenkanüle (2) und/oder die Innennadel (3) und das zweite Verbindungselement (9) über das Fixierelement (10) miteinander verklemmbar sind.

2. Biopsienadel nach Anspruch 1,
**dadurch gekennzeichnet,**
**daß** das Fixierelement (10) integral mit bzw. nicht abnehmbar von dem zweiten Verbindungselement (9) ausgebildet ist, insbesondere daß das Fixierelement (10) mit dem zweiten Verbindungselement verklebt ist.

3. Biopsienadel nach Anspruch 1 oder 2,
**dadurch gekennzeichnet,**
**daß** die Innennadel (3) und die Außenkanüle (2) und/oder die Innennadel (3) und das zweite Verbindungselement (9) im wesentlichen senkrecht zur Längserstreckung der Innennadel (3) zumindest bereichsweise gegeneinander verschiebbar sind, und/oder daß das Fixierelement (10) eine Durchgangsöffnung (19), insbesondere eine Bohrung, zur Durchführung der Innennadel (3) besitzt und daß zur Verklemmung von Innennadel (3) und Außenkanüle (2) die Innennadel (3) durch das Fixierelement (10) gegen die Innenwand der Außenkanüle (2) und/oder gegen die Innenwand der Durchgangsöffnung (19) preßbar ist.

4. Biopsienadel nach einem der vorhergehenden Ansprüche,
**dadurch gekennzeichnet,**
**daß** das Fixierelement (10) eine Drehbetätigung (11) umfaßt, die um eine im wesentlichen senkrecht zur Längsachse der Außenkanüle (2) verlaufende Drehachse (12) verdrehbar ist, wodurch ein mit der Drehbetätigung (11) verbundenes Klemmelement (21) entlang der Drehachse (12) verschiebbar ist, insbesondere daß das Klemmelement (21) eine Durchgangsöffnung (22), insbesondere eine Bohrung, zum Durchführen der Innennadel (3) besitzt und über ein Gewinde (23, 24) mit der Drehbetätigung (11) verbunden ist, und/oder daß die Drehbetätigung (11) zwischen zwei Anschlägen (46) verstellbar ist, wobei der eine Anschlag die Fixierstellung und der andere Anschlag (46) die Freigabestellung definiert.

5. Biopsienadel nach Anspruch 4,
**dadurch gekennzeichnet,**
**daß** die Drehbetätigung (11) eine länglich ausgebildete Handhabe (25) umfaßt, die in der Fixierstellung von Innennadel (3) und Außenkanüle (2) im wesentlichen parallel zur Außenkanüle (2) verläuft, insbesondere daß die Handhabe (25) in der Freigabestellung im wesentlichen senkrecht zur Außenkanüle (2) verläuft.

6. Biopsienadel nach Anspruch 5,
**dadurch gekennzeichnet,**
**daß** die Handhabe (25) einen Bereich, insbesondere einen Ansatz (14), aufweist, der zum verschiebbaren Einsetzen in eine Längsausnehmung (44) eines Gehäuseteils, insbesondere eines Deckels (29), des Nadelhalters (28) ausgebildet ist, insbesondere daß der Bereich bzw. der Ansatz (14) länglich ausgebildet ist und sich insbesondere in Längsrichtung der Handhabe (25) erstreckt.

7. Biopsienadel nach einem der vorhergehenden Ansprüche,
**dadurch gekennzeichnet,**
**daß** das erste Verbindungselement (7) zum Verrasten mit dem ersten Kopplungselement (33) ausgebildet ist und/oder daß das zweite Verbindungselement (9) zum Verrasten mit dem zweiten Kopplungselement (34) ausgebildet ist.

8. Biopsienadel nach einem der vorhergehenden Ansprüche,
**dadurch gekennzeichnet,**
**daß** das erste Verbindungselement (7) einen Anzeigeabschnitt (45) zur von außen ersichtlichen Anzeige seiner jeweiligen Position innerhalb des Nadelhalters (28) umfaßt, insbesondere daß der Anzeigeabschnitt als insbesondere länglicher Ansatz (45) ausgebildet ist, der zum verschiebbaren Einsetzen in eine Längsausnehmung (44) eines Gehäuseteils, insbesondere eines Deckels (29), des Nadelhalters (28) ausgebildet ist.

9. Biopsiesystem mit einer Biopsienadel (1) nach einem der Ansprüche 1 bis 8 und einem Nadelhalter (28) für die Biopsienadel (1), wobei der Nadelhalter (28) ein erstes und ein zweites Kopplungselement (33, 34) aufweist, wobei das erste Kopplungselement (33) mit dem an der Innennadel (3) vorgesehenen ersten Verbindungselement (7) und das zweite Kopplungselement (34) mit dem an der Außenkanüle (2) vorgesehenen zweiten Verbindungselement (9) verbindbar ist, und das erste und das zweite Kopplungselement (33, 34) zum Spannen des Nadelhalters (28) nacheinander jeweils gegen eine Federvorspannung verschiebbar sind, und wobei im ungespanntem Zustand des Nadelhalters (28) die Biopsienadel (1) bei gegeneinander unverschiebbar fixierter Innennadel (3) und Außenkanüle (2) sowohl in den Nadelhalter (28) einsetzbar als auch aus diesem entnehmbar ist.

10. Biopsiesystem nach Anspruch 9,
**dadurch gekennzeichnet,**
**daß** in einem ersten Schritt zum Teilspannen des Nadelhalters (28) das zweite Kopplungselement (34) und in einem darauffolgenden zweiten Schritt zum vollständigen Spannen des Nadelhalters (28) das erste Kopplungselement (33) gegen die Federvorspannung verschiebbar sind und/oder daß der Verschiebeweg des ersten und des zweiten Kopplungselements (33, 34) und damit die Eindringtiefe der Biopsienadel (1) kontinuierlich einstellbar ist.

11. Biopsiesystem nach Anspruch 9 oder 10,
**dadurch gekennzeichnet,**
**daß** zur Begrenzung der Verschiebewege für das erste und das zweite Kopplungselement (33, 34) jeweils Begrenzungsanschläge (49, 50) vorgesehen sind und daß die Begrenzungsanschläge (49, 50) insbesondere über ein Schraubgewinde kontinuierlich parallel zur Verschieberichtung der Kopplungselemente (33, 34) verstellbar sind, insbesondere daß im Bereich zwischen den Kopplungselementen (33, 34) und den Begrenzungsanschlägen (49, 50), insbesondere an den Begrenzungsanschlägen (49, 50), insbesondere elastische Dämpfungselemente zur Schall- und Aufpralldämpfung vorgesehen sind.

12. Biopsiesystem, nach einem der Ansprüche 9 bis 11,
**dadurch gekennzeichnet,**
**daß** an dem Nadelhalter (28) ein Anzeigeelement (51, 52, 53) zum Anzeigen der eingestellten Eindringtiefe vorgesehen ist und/oder daß zum Entspannen des Nadelhalters (28) und damit zum Einschießen der Biopsienadel (1) ein Druckknopf (56) an der der Austrittsstelle (58) der Biopsienadel (1) gegenüberliegenden Seite (55) des Nadelhalters (28) angeordnet ist.

13. Biopsiesystem nach einem der Ansprüche 9 bis 12,
**dadurch gekennzeichnet,**
**daß** der Nadelhalter (28) ein Gehäuse (30) mit einem Deckel (29) umfaßt und daß in dem Deckel (29) eine Längsausnehmung (44) zur Aufnahme und längsverschiebbaren Führung eines an dem zweiten Verbindungselement (9) vorgesehenen Spannungs-Anzeigeelements (14) ausgebildet ist und/oder daß der Nadelhalter (28) ein Gehäuse (30) mit einem Deckel (29) umfaßt und daß in dem Deckel (29) eine Längsausnehmung (44) zur Aufnahme und längsverschiebbaren Führung eines an dem ersten Verbindungselement (7) vorgesehenen Spannungs-Anzeigeelements (45) ausgebildet ist und/oder daß die Biopsienadel (1) auch im vollständig gespannten Zustand des Nadelhalters (28) in diesen einsetzbar und aus diesem entnehmbar ist.

## Claims

1. A biopsy needle for taking tissue samples for use in a needle holder (28), in particular in a biopsy gun, having an inner needle (3) which has a cut-out for the reception of the tissue sample to be taken in the region of its distal end and a first connection element (7) for connection to a first coupling element (33) of the needle holder (28) in the region of its proximal end (6), and having an outer cannula (2) in which the inner needle (3) is longitudinally displaceably supported and which has a second connection element (9) for connection to a second coupling element (34) of the needle holder (28) in the region of its proximal end (8), said second connection element (9) including a fixing element (10) which is adjustable between a fixing position and a release position, wherein the inner needle (3) and the outer cannula (2) are mutually longitudinally displaceable in the release position and the inner needle (3) and the outer cannula (2) are fixed mutually non-displaceably in the fixing position,
**characterised in that**
the inner needle (3) and the outer cannula (2) and/or the inner needle (3) and the second connection element (9) can be mutually clamped via the fixing element (10).

2. A biopsy needle in accordance with claim 1,
**characterised in that**
the fixing element (10) is made integral with or not removable from the second connection element (9); in particular **in that** the fixing element (10) is adhesively bonded to the second connection element.

3. A biopsy needle in accordance with claim 1 or claim 2,
**characterised in that**
the inner needle (3) and the outer cannula (2) and/or the inner needle (3) and the second connection element (9) are at least regionally mutually displaceable substantially perpendicular to the longitudinal extent of the inner needle (3); and/or **in that** the fixing element (10) has a passage opening (19), in particular a bore, for leading through the inner needle (3); and **in that** the inner needle (3) can be pressed by the fixing element (10) toward the inner wall of the outer cannula (2) and/or toward the inner wall of the passage opening (19) for clamping the inner needle (3) and the outer cannula (2).

4. A biopsy needle in accordance with any one of the preceding claims,
**characterised in that**
the fixing element (10) includes a rotary actuator (11) which is rotatable about an axis of rotation (12) extending substantially perpendicular to the longitudinal axis of the outer cannula (2), whereby a clamping element (21) connected to the rotary actuator (11) is displaceable along the axis of rotation (12); in particular **in that** the clamping element (21) has a passage opening (22), in particular a bore, for leading through the inner needle (3) and is connected to the rotary actuator (11) via a thread (23, 24); and/or **in that** the rotary actuator (11) is adjustable between two abutments (46), with the one abutment defining the fixing position and the other abutment (46) defining the release position.

5. A biopsy needle in accordance with claim 4,
**characterised in that**
the rotary actuator (11) includes a handle (25) which is of elongate form and which extends substantially parallel to the outer cannula (2) in the fixing position of the inner needle (3) and the outer cannula (2); in particular **in that** the handle (25) extends substantially perpendicular to the outer cannula (2) in the release position.

6. A biopsy needle in accordance with claim 5,
**characterised in that**
the handle (25) has a region, in particular a protrusion (14), which is designed for displaceable insertion into a longitudinal cut-out (44) of a housing part, in particular of a cover (29), of the needle holder (28); in particular **in that** the region or the protrusion (14) is of longitudinal form and in particular extends in the longitudinal direction of the handle (25).

7. A biopsy needle in accordance with any one of the preceding claims,
**characterised in that**
the first connection element (7) is designed for latching to the first coupling element (33); and/or **in that** the second connection element (9) is designed for latching to the second coupling element (34).

8. A biopsy needle in accordance with any one of the preceding claims,
**characterised in that**
the first connection element (7) includes a display section (45) for the outwardly visible display of its respective position within the needle holder (28); in particular **in that** the display section is designed as a protrusion (45), in particular as an elongate protrusion, which is designed for displaceable insertion into a longitudinal cut-out (44) of a housing part, in particular of a cover (29), of the needle holder (28).

9. A biopsy system having a biopsy needle (1) in accordance with any one of the claims 1 to 8 and having a needle holder (28) for the biopsy needle (1), wherein the needle holder (28) has a first coupling element and a second coupling element (33, 34), wherein the first coupling element (33) can be connected to the first connection element (7) provided at the inner needle (3) and the second coupling element (34) can be connected to the second connection element (9) provided at the outer cannula (2) and the first and second coupling elements (33, 34) are displaceable after one another in each case against a spring preload for loading the needle holder (28), and wherein the biopsy needle (1) can be both inserted into and removed from the needle holder (28) in the non-loaded state of the needle holder (28) with a mutually non-displaceably fixed inner needle (3) and outer cannula (2).

10. A biopsy system in accordance with claim 9,
**characterised in that**
the second coupling element (34) is displaceable against the spring preload in a first step for part-loading the needle holder (28) and the first coupling element (33) is displaceable against the spring preload in a subsequent second step for fully loading the needle holder (28); and/or **in that** the displacement path of the first and second coupling elements (33, 34), and thus the penetration depth of the biopsy needle (1), can be continuously adjusted.

11. A biopsy system in accordance with claim 9 or claim 10,
**characterised in that**
respective limiting abutments (49, 50) are provided to limit the displacement paths for the first and second coupling elements (33, 34); and **in that** the limiting abutments (49, 50) can be continuously adjusted parallel to the direction of displacement of the coupling elements (33, 34), in particular via a screw thread; and in particular in that damping elements, in particular elastic damping elements, for the damping of noise and impact are provided in the region between the coupling elements (33, 34) and the limiting abutments (49, 50), in particular at the limiting abutments (49, 50).

12. A biopsy system in accordance with any one of the claims 9 to 11,
**characterised in that**
a display element (51, 52, 53) for displaying the set penetration depth is provided at the needle holder (28); and/or **in that** a push button (56) is arranged at the side (55) of the needle holder (28) disposed opposite the outlet point (58) of the biopsy needle (1) for unloading the needle holder (28) and thus for injecting the biopsy needle (1).

13. A biopsy system in accordance with any one of the claims 9 to 12,
**characterised in that**
the needle holder (28) includes a housing (30) having a cover (29); and **in that** a longitudinal cut-out (44) is formed in the cover (29) for the reception and longitudinally displaceable guidance of a load display element (14) provided at the second connection element (9); and/or **in that** the needle holder (28) includes a housing (30) having a cover (29); and **in that** a longitudinal cut-out (44) for the reception and longitudinally displaceable guidance of a load display element (45) provided at the first connection element (7) is formed in the cover (29); and/or **in that** the biopsy needle (1) can also be inserted into and removed from the needle holder (28) in the completely loaded state.

## Revendications

1. Aiguille de biopsie destinée au prélèvement d'échantillons de tissus, à utiliser dans un porte-aiguille (28), en particulier un "pistolet" de biopsie, comprenant une aiguille intérieure (3) qui comprend, dans la région de son extrémité distale, un évidement pour la réception de l'échantillon de tissu à prélever et, dans la région de son extrémité proximale (6), un premier élément de liaison (7) à relier avec un premier élément de couplage (33) du porte-aiguille (28), et comprenant une canule extérieure (2) dans laquelle l'aiguille intérieure (3) est montée avec possibilité de translation longitudinale et qui comprend, dans la région de son extrémité proximale (8), un second élément de liaison (9) à relier avec un second élément de couplage (34) du porte-aiguille (28), ledit second élément de liaison (9) comprenant un élément de fixation (10) qui est déplaçable entre une situation de fixation et une situation de libération, de sorte que dans la situation de libération l'aiguille intérieure (3) et la canule extérieure (2) sont déplaçables longitudinalement l'une par rapport à l'autre et que dans la situation de fixation l'aiguille intérieure (3) et la canule extérieure (2) sont fixées de manière non déplaçable l'une par rapport à l'autre,
**caractérisée en ce que**
l'aiguille intérieure (3) et la canule extérieure (2) et/ou l'aiguille intérieure (3) et le second élément de liaison (9) sont susceptibles d'être coincé(e)s l'une avec l'autre via l'élément de fixation (10).

2. Aiguille de biopsie selon la revendication 1,
**caractérisée en ce que** l'élément de fixation (10) est réalisé de manière intégrale avec ou de manière non démontable depuis le second élément de liaison (9), en particulier **en ce que** l'élément de fixation (10) est collé avec le second élément de liaison.

3. Aiguille de biopsie selon la revendication 1 ou 2,
**caractérisée en ce que** l'aiguille intérieure (3) et la canule extérieure (2) et/ou l'aiguille intérieure (3) et le second élément de liaison (9) sont déplaçables l'une par rapport à l'autre au moins localement et sensiblement perpendiculairement à l'extension longitudinale de l'aiguille intérieure (3), et/ou **en ce que** l'élément de fixation (10) possède une ouverture traversante (19), en particulier un perçage, pour la traversée de l'aiguille intérieure (3), et **en ce que** pour le coincement de l'aiguille intérieure (3) et de la canule extérieure (2), l'aiguille intérieure (3) est susceptible d'être pressée par l'élément de fixation (10) contre la paroi intérieure de la canule extérieure (2) et/ou contre la paroi intérieure de l'ouverture traversante (19).

4. Aiguille de biopsie selon l'une des revendications précédentes,
**caractérisée en ce que** l'élément de fixation (10) comprend un organe d'actionnement rotatif (11), qui est capable de tourner autour d'un axe de rotation s'étendant sensiblement perpendiculairement à l'axe longitudinal de la canule extérieure (2), en raison de quoi un élément de coincement (21) relié à l'organe d'actionnement rotatif (11) est déplaçable le long de l'axe de rotation (12), en particulier **en ce que** l'élément de coincement (21) possède une ouverture traversante (22), en particulier un perçage, pour la traversée de l'aiguille intérieure (3) et est relié via un pas de vis (23, 24) avec l'organe d'actionnement rotatif (11), et/ou **en ce que** l'organe d'actionnement rotatif (11) est déplaçable entre deux butées (46), l'une des butées définissant la situation de fixation et l'autre butée (46) définissant la situation de libération.

5. Aiguille de biopsie selon la revendication 4,
**caractérisée en ce que** l'organe d'actionnement rotatif (11) comprend une manette (25) de réalisation allongée qui, dans la situation de fixation de l'aiguille intérieure (3) et de la canule extérieure (2), s'étend sensiblement parallèlement à la canule extérieure (2), en particulier **en ce que** la manette (25) s'étend, dans la situation de libération, essentiellement perpendiculairement à la canule extérieure (2).

6. Aiguille de biopsie selon la revendication 5,
**caractérisée en ce que** la manette (25) comprend une région, en particulier un talon (14), qui est réalisée pour être mis en place en translation dans un évidement longitudinal (44) d'une partie de boîtier, en particulier d'un couvercle (29), du porte-aiguille (28), en particulier **en ce que** la région ou le talon (14) est de réalisation allongée et s'étend en particulier en direction longitudinale de la manette (25).

7. Aiguille de biopsie selon l'une des revendications précédentes,
**caractérisée en ce que** le premier élément de liaison (7) est réalisé pour venir s'enclencher avec le premier élément de couplage (33) et/ou **en ce que** le second élément de liaison (9) est réalisé pour venir s'enclencher avec le second élément de couplage (34).

8. Aiguille de biopsie selon l'une des revendications précédentes,
**caractérisée en ce que** le premier élément de liaison (7) comprend un tronçon d'affichage (45) destiné à afficher de manière visible de l'extérieur sa position respective à l'intérieur du porte-aiguille (28), en particulier **en ce que** le tronçon d'affichage est réalisé comme un talon (45), en particulier allongé qui est réalisé pour être mis en place en translation dans un évidement allongé (44) d'une partie de boîtier, en particulier d'un couvercle (29) du porte-aiguille (28).

9. Système de biopsie comprenant une aiguille de biopsie (1) selon l'une des revendications 1 à 8 et un porte-aiguille (28) pour l'aiguille de biopsie (1), dans lequel le porte-aiguille (28) comprend un premier élément de couplage et un second élément de couplage (33, 34), le premier élément de couplage (33) pouvant être relié avec le premier élément de liaison (7) prévu sur l'aiguille intérieure (3) et le second élément de couplage (34) pouvant être relié avec le second élément de liaison (9) prévu sur la canule extérieure (2), et le premier et le second élément de couplage (33, 34) sont déplaçables, pour serrer le porte-aiguille (28), l'un après l'autre respectivement contre la précontrainte d'un ressort et, dans la situation non tendue du porte-aiguille (28), l'aiguille de biopsie (1) peut être aussi bien mise en place dans le porte-aiguille (28) qu'être enlevée hors de celui-ci lorsque l'aiguille intérieure (3) et la canule extérieure (2) sont fixées de manière indéplaçable l'une par rapport à l'autre.

10. Système de biopsie selon la revendication 9,
**caractérisé en ce que**, dans une première étape pour serrer partiellement le porte-aiguille (28) le second élément de couplage (34) est déplaçable à l'encontre de la précontrainte du ressort et, dans une seconde étape successive, pour serrer totalement le porte-aiguille (28), le premier élément de couplage (33) est déplaçable à l'encontre de la précontrainte du ressort, et/ou **en ce que** le trajet de déplacement du premier et du second élément de couplage (33, 34) et ainsi la profondeur de pénétration de l'aiguille de biopsie (1), est réglable en continu.

11. Système de biopsie selon la revendication 9 à 10,
**caractérisé en ce que** pour limiter les trajets de déplacement pour le premier et le second élément de couplage (33, 34) il est prévu des butées de limitation respectives (49, 50), et **en ce que** les butées de limitation (49, 50) sont déplaçables, en particulier via un pas de vis, de manière continue parallèlement à la direction de déplacement des éléments de couplage (33, 34), en particulier **en ce que** des éléments d'amortissement, en particulier élastiques et destinés à l'amortissement des bruits et des impacts, sont prévus dans la région entre les éléments de couplage (33, 34) et les butées de limitation (49, 50), en particulier contre les butées de limitation (49, 50).

12. Système de biopsie selon l'une des revendications 9 à 11,
**caractérisé en ce qu'**un élément d'affichage (51, 52, 53) destiné à l'affichage de la profondeur de pénétration réglée est prévu sur le porte-aiguille (28) et/ou **en ce qu'**un bouton-poussoir (56) est agencé sur le côté (55) du porte-aiguille (28) opposé à l'emplacement de sortie (58) de l'aiguille de biopsie (1) pour desserrer le porte-aiguille (28) et donc pour projeter l'aiguille de biopsie (1).

13. Système de biopsie selon l'une des revendications 9 à 12,
**caractérisé en ce que** le porte-aiguille (28) comprend un boîtier (30) avec un couvercle (29), et **en ce qu'**un évidement allongé (44) est réalisé dans le couvercle (29) pour recevoir et pour guider en déplacement longitudinal un élément d'affichage de serrage (14) prévu sur le second élément de liaison (9), et/ou **en ce que** le porte-aiguille (28) comprend un boîtier (30) avec un couvercle (29), et **en ce qu'**un évidement allongé (44) est réalisé dans le couvercle (29) pour recevoir et pour guider en déplacement longitudinal un élément d'affichage de serrage (45) prévu sur le premier élément de liaison (7), et/ou **en ce que** l'aiguille de biopsie (1) peut être mise en place dans le porte-aiguille (28) et être enlevée hors de celui-ci également dans la situation totalement serrée du porte-aiguille (28).
